# EUROPEAN PATENT APPLICATION

(11) **EP 2 166 507 A1**
(43) Date of publication of application: **24.03.2010**
(21) Application number: 08765633.6
(22) Date of filing: 16.06.2008
(51) Int. Cl.: G06Q 50/00

(54) **MANAGEMENT SYSTEM FOR PRESCRIBING/PREPARING MEDICINE WHICH MAY CAUSE SEVERE SIDE EFFECT**

(30) Priority: 21.06.2007 JP 2007163461
(71) Applicant: Fujimoto Co., Ltd., Matsubara-shi Osaka 580-0011 (JP)
(72) Inventor: FUJIMOTO, Muneya, Osaka-shi Osaka 545-0001 (JP); HASE, Masafumi, Matsubara-shi Osaka 580-0011 (JP); KITAKOJI, Junichi, Matsubara-shi Osaka 580-0011 (JP); NAKAO, Chikako, Matsubara-shi Osaka 580-0011 (JP); IWAKI, Kenichi, Matsubara-shi Osaka 580-0011 (JP); SABATO, Takaaki, Matsubara-shi Osaka 580-0011 (JP)
(74) Representative: Calderbank, Thomas Roger
(86) International application number: PCT/JP2008/060950
(87) International publication number: WO 2008/156045

(57) **Abstract**

There is provided a medicine management system in which, in order to prevent a medicine known as the one which may cause a serious side effect when a certain patient takes it from being transferred to the certain patient, it is judged whether or not information from the prescribing doctor, the patient, and the pharmacist all registered in the system respectively satisfies a transfer condition, the result is notified, and records can be preserved for individual information providers not by telephone and IVRs.

The medicine management system comprises input means, distinction means, transmitting means and an information storage medium as described in the following (1) to (6):
(1) input means for inputting a first management data aggregate including a first confirmation format including a plurality of confirmation items between a doctor and a patient for avoiding a serious side effect to a medicine and check columns corresponding thereto; and answers recorded therein, in response to transmission from a doctor;
(2) distinction means (i) to (iii) for data in the first management data aggregate;
(3) transmitting means for producing an integrated confirmation format in which a second confirmation format including a plurality of confirmation items between a pharmacist and a patient and check columns corresponding thereto is appended in addition to the first management data aggregate or in addition to a prescription description recorded by a doctor, which is the data selected from the first management data aggregate, only in case of distinction as conformance in the distinction according to (2), and replying the integrated confirmation format to the doctor or the pharmacist;
(4) input means for inputting an integrated confirmation format, in which a second management data aggregate including a recorded answer between a pharmacist and a patient in the second confirmation format is appended to, in response to transmission from the pharmacist;
(5) distinction means (i) to (iii) for data in the second management data aggregate; and
(6) transmitting means for replying judgment of the propriety of dispensing depending on the distinction result to the pharmacist.

## Description

### Technical Field

The present invention relates to management systems for preventing medicines known as the ones which may cause serious side effects when patients under certain conditions such as pregnancy and contraindications for coadministration take them from being transferred to the patients. More specifically, the present invention relates to a medicine management system, in which it is distinguished and judged whether or not information from the prescribing doctor, the patient and the pharmacist all registered in the system respectively satisfies a transfer condition, and the doctor and/or the pharmacist are notified of the result.

### Background Art

Medicines generally have undesirable side effects with varying degrees of severity. Side effects include: ones developed in most patients receiving treatment although their symptoms are mild, e.g., constipation and nausea; and ones as being fatal depending on the states of patients. In addition, there are side effects developed only in patients with certain conditions such as pregnancy and contraindications for coadministration. In the case of such a side effect as to occur when a patient is applicable to such a certain condition, it is extremely important to prevent the patient applicable to the condition from taking a medicine or to prevent the patient from falling into such a state to avoid damage from the side effect.

Representative medicines necessary for avoiding damage from such a side effect include thalidomide. Thalidomide is known to be effective for treatment of multiple myeloma and the like while it is a teratogenic drug which affects fetal development in the mother by being taken by the pregnant woman. Accordingly, pregnant women or patients suspected of being pregnant should avoid taking teratogenic drugs. It is also important to prevent damage by accidental ingestion of such a drug, and the like, and taking of a medicine in accordance with a well-established medicine management system is thus desired.

Therefore, US 6045501 discloses a method for delivering a medicine to a patient while avoiding occurrence of a harmful side effect known to occur or suspected of occurring by the medicine, in which the medicine is prescribed only after confirmation that a prescriber is registered in a storage medium which is readable by a computer and is qualified to prescribe the medicine, a pharmacy is registered in the medium and is qualified to provide a prescription for the medicine, and a patient is registered in the medium and is approved to receive the medicine.

In addition, as a modification of US 6045501, National Publication of International Patent Application No. 2004-512617 discloses a method, in which a prescription approval code that will not be provided unless prescribers, pharmacies, patients, a risk group in the patients, and the informed consents of the patients are properly registered in the storage medium is set and an effort required of the registered pharmacies is minimized and simplified by searching the prescription approval code by the pharmacies.

Based on US 6045501 and National Publication of International Patent Application No. 2004-512617 as described above, a system S.T.E.P.S. for safety management of thalidomide has been operated in the U. S. In the S.T.E.P.S., doctors dealing with the medicine, patients and pharmacists are registered in a central center for uniform management. Furthermore, at regular intervals, the central center is to perform investigation just after receiving the offer of information from the patients, prescription descriptions and other information (management on a transfer condition) from the doctors by prescriptions, and the confirmation reports of prescription descriptions and their dispensing information from the pharmacists, to perform safety management of medicines. The neglect of the offer of the information to the center by the doctors, the patients and the pharmacists will preclude prescription and dispensing, and thus make it impossible to maintain treatment. In the S.T.E.P.S., the offer of the information is performed using an interactive voice response system (IVR) with telephone.

However, in this case, no answer description at the time is recorded in a side receiving the offer of the information/investigation with telephone, and therefore no evidence with a probative value for the occurrence of a problem is accumulated. In response with telephone, an awareness about strict compliance with rules will be also decreased in persons concerned.

In addition, Japanese Patent Laid-Open No. 2006-285973 discloses a medicine management system, in which the protection of personal information is planned by access confinement means for confining access to prescription information and patient information by certain persons, the information of the management state of a medicine after handing the medicine to a patient who is more likely to deviate from rules than a doctor and a pharmacist is fed back by recovering a container for exclusive use of the medicine handed to the patient, and therefore management according to the patient is enabled by being provided with means for comparing information of taking a medicine by the patient with medicine history information based on prescription information.

[Patent Document 1] US 6045501
[Patent Document 2] National Publication of International Patent Application No. 2004-512617
[Patent Document 3] Japanese Patent Laid-Open No. 2006-285973

### Disclosure of the Invention

### Problems to be Solved by the Invention

No answer description is surely recorded to result in a problematic probative value in the system with telephone whereas, in oral response, awareness about strict compliance with rules is decreased in persons concerned. Thus, when many people are involved in operation of a management system, it is important to clearly define the responsibilities of the respective people, so that, generally, events are recorded, the signatures of the respective people are affixed to the records, and the records are stored. Accordingly, the construction of a system in which this practice is maintained is desired.

An object of the invention is to provide a medicine management system in which, in order to prevent a medicine known as the one which may cause a serious side effect when a certain patient takes it from being transferred to the certain patient, it is judged whether or not information from the prescribing doctor, the patient, and the pharmacist all registered in the system respectively satisfies a transfer condition, the result is notified, and records can be kept for individual information providers not by telephone and IVRs.

### Means for Solving the Problems

The present invention is a system, in which the offer of information can be recorded and preserved for a person concerned, wherein the offer of information is performed by the person concerned using visible means such as a fax or digital paper with a special-purpose format for offering information, it is distinguished and judged whether or not the information satisfies a transfer condition by processing on a program, and information providers are notified of the judgment result.

More specifically, the present invention is to provide a medicine management system comprising input means, distinction means, transmitting means and an information storage medium as described in the following (1) to (6):
(1) input means for inputting a first management data aggregate including a first confirmation format including a plurality of confirmation items between a doctor and a patient for avoiding a serious side effect to a medicine and check columns corresponding thereto; and answers recorded therein, in response to transmission from a doctor;
(2) distinction means (i) to (iii) described below for data in the first management data aggregate:
   (i) distinction means for distinguishing whether or not a doctor and a patient are persons registered previously in an information storage medium for dealing with said medicine;
   (ii) distinction means for distinguishing whether or not each piece of patient data conforms to a medicine prescription requirement according to a patient group; and
   (iii) distinction means for distinguishing whether or not medicine prescription interval and prescribed medicine quantity data is within an appropriate numerical range;
(3) transmitting means for producing an integrated confirmation format in which a second confirmation format including a plurality of confirmation items between a pharmacist and a patient and check columns corresponding thereto is appended in addition to the first management data aggregate or in addition to a prescription description recorded by a doctor, which is the data selected from the first management data aggregate, only in case of distinction as conformance in the distinction according to (2), and replying the integrated confirmation format to the doctor or the pharmacist;
(4) input means for inputting an integrated confirmation format, in which a second management data aggregate including a recorded answer between a pharmacist and a patient in the second confirmation format is appended, in response to transmission from the pharmacist;
(5) distinction means (i) to (iii) described below for data in the second management data aggregate:
   (i) distinction means for distinguishing whether or not a patient in the second management data aggregate is the same as a patient in the first management data aggregate;
   (ii) distinction means for distinguishing whether or not a pharmacist is a person registered previously in an information storage medium for dealing with said medicine; and
   (iii) distinction means for distinguishing whether or not dispensing quantity data in the second management data aggregate conforms to prescription data in the first management data aggregate; and
(6) the transmitting means for replying judgment of the propriety of dispensing depending on the distinction result to the pharmacist.

Medicines targeted in accordance with the present invention, which are prescription pharmaceuticals used under a prescription from a doctor, include, but are not particularly limited to, prescription pharmaceuticals such as analgetics, antiphlogistics, sympathomimetics, cardiotonics, antibiotics, antiallergic agents, antihistaminics, bronchodilators, antitumor agents, antiarrhythmic agents, anticonvulsants, antiparkinson agents, antihypertensive agents, centrally acting agents, antidiabetic agents, gastrointestinal agents, cholinergic agents and antiviral agents, more specifically, morphine, hydromorphone, oxycodone, methadone, meperidine, dihydrocodeine, codeine, amphetamine, dihydromorphine, buprenorphine, fentanyl, naproxen, isopropylantipyrine, ibuprofen, ketoprofen, diclofenac, ephedrine, selegiline, salbutamol, terbutaline, phenyl propanolamine, pheniramine, terfenadine, chlorpheniramine, diphenhydramine, clemastin, procainamide, propranolol, quinidine, metoprolol, captopril, hydralazine, diltiazem, amoxicillin, cephalexin, clarithromycin, penicillin, cloxacillin sodium, metronidazole, theophyline, salbutamol, flutamide, thalidomide, fluorouracil, procainamide, quinidine, phenytoinsodium, ethosuximide, sodium valproate, diazepam, perphenazine, chlorpromazine, famotidine, ranitidine, rametidine, omeprazole, lansoprazole, tolbutamide, acarbose, voglibose, bethanechol, neostigmine, carbachol, sorivudine, vitamins, amino acids, peptides, and the like; and inorganic salts or organic salts, such as hydrochlorides, sulfates, phosphates, fumarates, maleates, tartrates and bitartrates thereof, and particularly are desirably applied to pharmaceuticals belonging to narcotics, psychotropic agents, stimulants, stimulants raw materials, powerful drugs, poisonous drugs, and the like; pharmaceuticals which are intended to be used for cancer or other specific diseases and known to have serious side effects; pharmaceuticals requiring strict management, such as pharmaceuticals that are contraindicated for coadministration due to serious interactions; and pharmaceuticals with high risks of creating hazards unless being used properly under the guidance of a doctor or a pharmacist. The side effects in accordance with the present invention, which mean all undesirable reactions caused by medicines, are not limited only to reactions to patients themselves who take the medicines but also include side effects, such as teratogenicity causing serious malformations in the embryos of the patients, which may occur across generational lines.

A doctor who prescribes the medicine in accordance with the present invention is a doctor having sufficient knowledge of use of a prescription pharmaceutical, prescribes the pharmaceutical for a patient, and is registered in the system (hereinafter may be referred to as a prescribing doctor), and transmission to the system may be performed by a person who representatively assists the work of the doctor under the direction of the doctor, such as a nurse. The prescribing doctor provides education and guidance on dosage and signature, special precautions for use, and side effects to patients, their family members and helpers, and further explores the level of understanding of the items by the patients, etc., during medical practice. Information of such a doctor includes the registration number, medical institution name, address, full name, position, job title, URL, e-mail address, telephone number, recovery record, fax number, etc., of the doctor.

In accordance with the present invention, a pharmacist who dispenses a drug is a pharmacist who deals with a prescription pharmaceutical in a pharmacy in a medical institution or a pharmacy out of a hospital and is registered in the system ("primary pharmacist"; hereinafter may be simply referred to as "pharmacist"), and transmission to the system may be performed by a person who representatively assists the work of the pharmacist under the direction of the pharmacist. Information of a pharmacist includes the registration number, medical institution name, address, full name, position, job title, receipt and disbursement information of a prescription pharmaceutical, dispensing record, recovery record, URL, e-mail address, telephone number, fax number, etc., of the pharmacist.

A patient in accordance with the present invention is a registered patient who receives treatment using the system. Information of such a patient includes the registration number, name, the date of birth, blood type, the presence or absence of allergies, age, sex, address, e-mail address, telephone number, fax number, health insurance card number, name of disease, and diagnosis result of the patient, information of indication of taking a medicine, such as dosing days or a dosing amount, information of a medicine storage area, information of a practical medicine dosing amount and a remaining amount from a container for exclusive use of a medicine, medical history, family member information such as family structure or names, helper information, information about the level of understanding of another mode which is variously required depending on the medicine, and information about any deviation history, such as a case of no compliance with indicated dosage and signature or a case of loss of the medicine or its transfer to a third person.

The medicine management system of the present invention includes: registration means for inputting/storing information about prescription, information about a doctor to prescribe a medicine, information about a pharmacist to dispense a medicine and patient information; searching means for searching registered information; and data displaying means for displaying search results. As a management for such a medicine management system (hereinafter referred to as a management center), a prescription pharmaceuticals manufacturer or an institution to which a prescribing doctor or a pharmacist dispensing a medicine belong may be desirable.

When the medicine management system of the present invention is used, at least one computer and at least one access terminal are set up, network environments such as the Internet, an intranet and a LAN may be used, and the information may be transmitted using a memory medium such as a memory card storing the information, while the system is preferably utilized after inputting and registering information provided in a visual medium such as paper by means such as a fax and digital paper into the medicine management system of the present invention including well-known input means, distinction means, transmitting means and an information storage medium.

When the network environments are constructed, various conventional technologies, such as a firewall used as means for information security for restriction of access to the information, information encryption, and personal authentication by password-based memory verification (ID number, password, etc.), possession verification (key, IC card, hardware token, etc.) and biometric verification (fingerprint, retina/iris, face form, handwriting, DNA, vein pattern, voice, pinna, shape of palm, etc.), may be combined.

In accordance with an embodiment of the present invention, the first and second confirmation formats may be printed on paper called digital paper as described below to make an entry in the confirmation formats using a digital pen and to use the formats for transmission to and reception from a doctor or a pharmacist. Recently, a system which can directly digitize handwritten characters and graphics using digital paper which is special-purpose paper, on which small dot patterns are printed, which has been developed by Anoto Group AB of Sweden, and a digital pen to automatically digitize handwriting data has been put to practical use. As an application of the system, for example, a system, which enables integral management by a single system while making a conventional document medical chart and a new electronic chart coexist by making a required entry in a medical chart composed of digital paper by a digital pen to make a document medical chart, concurrently performing data entry of an electronic chart by making an entry in the medical chart, storing the document medical chart in a management depository of which it can be randomly taken out, and storing electronic chart data in a memory device for storage, is known (Japanese Patent Laid-Open No. 2005-141537).

A medical service support system, in which medical service-related data is input into a terminal by making an entry in digital paper provided with an input format into which data about medical service can be input using a digital pen, wherein a number line into which the value of the medical service-related data can be input is indicated in the input format, and conversion information storage means is associated with a written position on the number line to store the medical service-related data value, is also proposed (Japanese Patent Laid-Open No. 2006-178839).

### Advantages of the Invention

In accordance with the present invention, a pharmacist uses a predetermined format, in which information about a prescribing doctor and a patient is described, which is sent from a management center every dispensing, and therefore there is not required confirmation whether the prescribing doctor and the patient are registered, and the effort therefor needed in the pharmacist can be decreased. Use of such a special-purpose format also enables FAX-OCR processing. In the case of a special-purpose format made to be digital paper, each entry by a person concerned also becomes an input into a memory medium by making an entry using a digital pen, so that subsequent processing can be made to proceed without input operation by a person. In the present invention, the validity of the compliance statuses of a plurality of confirmation items which a doctor and a patient and/or a pharmacist and a patient should previously comply with is judged by a check column confirmed mutually by a doctor, a pharmacist and a patient in a special-purpose format without using an IVR system, any deviation from a compliance rule can be checked beforehand. In addition, since an answer description is stored as recording paper each time, the responsibility of a person to be involved is clarified, and the safety management of a medicine which may cause a serious side effect can be more surely performed.
When an integrated confirmation format in which a second confirmation format is appended to a prescription description, recorded by a doctor, which is data selected from a first management data aggregate, is used, a description space can be effectively used, a character is easily read, and mutual confirmation by a doctor, a pharmacist and a patient is surely performed, compared to an integrated confirmation format in which it is appended to a complete first management data aggregate.

An integrated confirmation format (including first and second confirmation formats) to be replied to a doctor or a pharmacist contains, as confirmation items for a pharmacist and a patient, items about whether or not the medicine is transferred to a third person, a storage status, and use and recovery of a container for exclusive use of the medicine, and can therefore make quantity management of the medicine surer by confirming the history of deviation from instruction items to take the medicine in the case of missing of the medicine, simultaneous taking of the medicine, transfer to another person, loss, theft, etc.
In addition, the input format further includes items about the quantity of a not-yet-taken medicine and a prescription or dispensing quantity, and therefore the quantity management of the medicine can be surely performed by prescribing and dispensing only a necessary quantity, in which the quantity of the not-yet-taken medicine is subtracted, to a patient second times or later.

Furthermore, the input format includes each signature column of a prescribing doctor, a patient and/or a pharmacist, and therefore medicine management can be more surely performed by promoting an awareness that the actions of prescribing/dispensing a medicine which may cause a serious side effect and taking it are based on self-responsibility.
Furthermore, when judging that prescription and dispensing are not appropriate due to the defectiveness of the input data, the predetermined format further provided with a printing space for indicating the defectiveness is made, appropriate data is retransmitted by transmitting the indication to the doctor and/or the pharmacist, and therefore the surer safety management of a medicine which may cause a serious side effect is enabled.

In accordance with conventional suggestions, no answer description at the time is recorded in a side receiving the offer of the information/investigation with telephone, and therefore no evidence with a probative value for the occurrence of a problem is accumulated. In response with telephone, an awareness about strict compliance with rules will be also decreased in persons concerned. As described above, when many people are involved in operation of a management system, it is important to clearly define the responsibilities of the respective people, and the construction of a medicine management system in which such general practices that events are recorded, the signatures of the respective people are affixed to the records, and the records are stored is desired in terms of sureness and reliability.
For the first and second confirmation formats, a confirmation format, which is separated into: (i) a section composed of codes (which may be numbers) indicating respective confirmation items and check columns corresponding thereto; and (ii) an attached sheet section indicating the detailed descriptions of the confirmation items corresponding to the codes, wherein the paper of the above-mentioned (ii) is attached to the (i), as well as a confirmation format in which confirmation items and check columns corresponding thereto are integrated, may be also available. In this case, in comparison with the integrated confirmation format, a description space can be effectively used to make characters easily readable, so that mutual confirmation among a doctor, a pharmacist and a patient is made to be surer.

### Best Mode for Carrying Out the Invention

In order to more specifically describe the present invention, embodiments in which thalidomide having a teratogenic side effect is a prescription drug are described below. These do not limit the present invention but, depending on a side effect known in each prescription drug, modifications to education, explanation, instruction items or guidance, actions to be taken for a side effect, etc., in taking a medicine, are easy according to common general technical knowledge in the art. Embodiments according to the present invention, in which transmission and reception by a doctor or a pharmacist are performed through a facsimile, are described referring to the drawings.

### Embodiment

### [Embodiment 1]

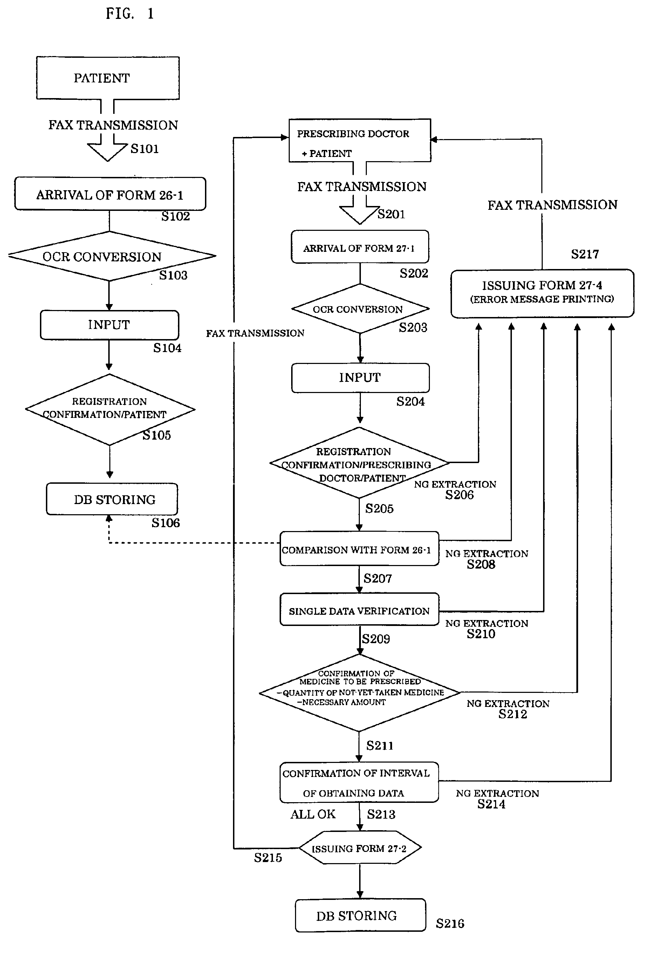

### (Prior to medical examination, see Figure 1)

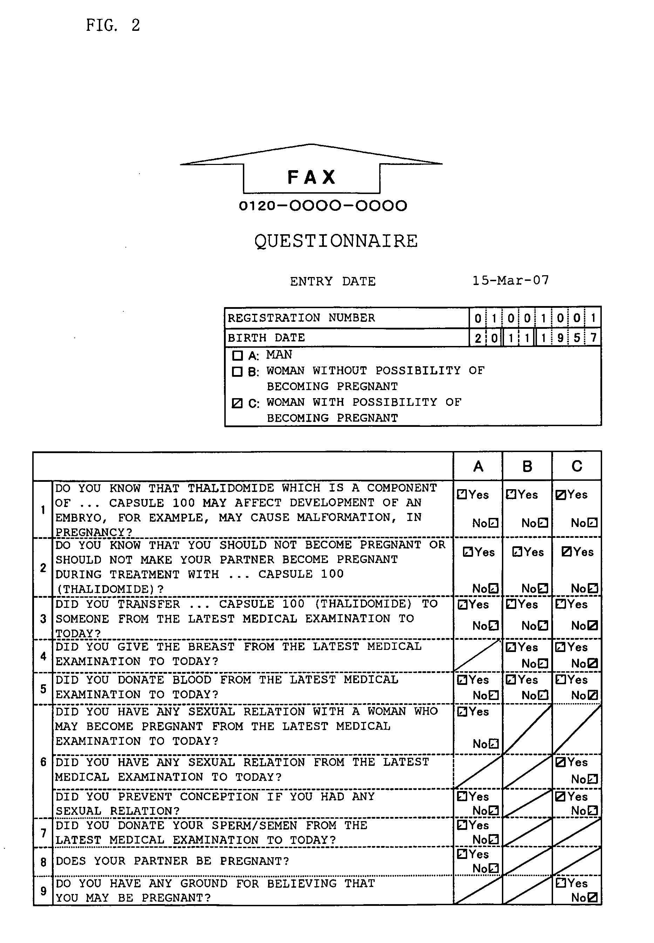

A patient answers the questions of a questionnaire (Form 26-1: Figure 2) prior to medical examination on the medical examination day and transmits the questionnaire to a management center by fax (Step 101). The management center uses OCR to convert the received questionnaire into data (Step 103). It is judged from the converted data whether or not the transmitting patient is registered (Step 105), and the data is stored in an information storage medium (Step 106).

### (In consultation room, see Figure 1)

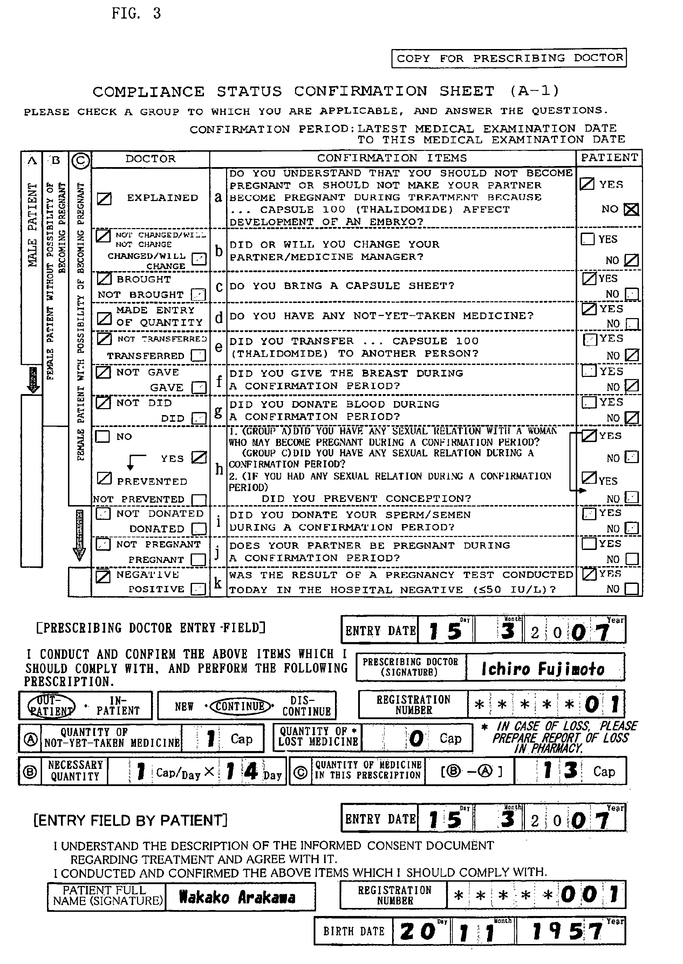

During medical examination, a prescribing doctor explains the medicine and safety management to the patient based on an informed consent document (not shown) regarding treatment attached to a first confirmation format (Form 27-1: Figure 3) and obtains his/her consent. The first confirmation format comprises a plurality of confirmation items between the doctor and the patient for avoiding the side effect of the medicine and check columns corresponding thereto and, in this embodiment, includes confirmation items (a) to (k) as shown in Figure 3. At a meeting, the patient and the prescribing doctor perform mutual confirmation every time prior to prescription according to each column of the confirmation items (a) to (k).

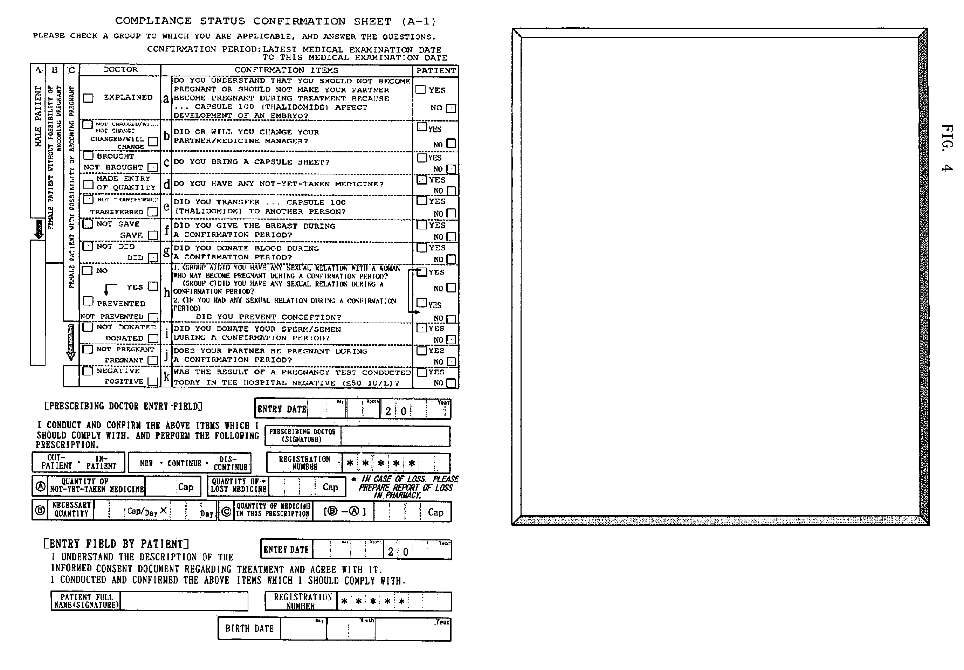

The patient makes entry of an entry date, a patient registration number and a patient birth date in the confirmation format, the prescribing doctor makes entry of the quantity of a medicine necessary until the next medical examination, the quantity of a not-yet-taken medicine, the quantity of a lost medicine in the case of occurrence of loss of the medicine, the quantity of this prescribed medicine taken into account the quantity of the medicine necessary until the next medical examination and the quantity of the not-yet-taken medicine, an entry date and a prescribing doctor registration number, and they sign in signature columns, respectively. The prescribing doctor transmits a first management data aggregate, in which answers are recorded and written in the first confirmation format in such a manner, to the management center by fax (Step 201). The management center uses OCR to convert the received first management data aggregate into data (Step 203). It is judged from the converted data whether or not the transmitting prescribing doctor and patient are registered previously in an information storage medium for dealing with the medicine (Step 205). In the case of an unregistered doctor or patient, the fact is printed in the printing space of the right column of Form 27-4 (Figure 4) to give an indication (Step 206).

The data of the questionnaire stored previously in the information storage medium is searched and compared with the received first management data aggregate, and a difference between the description answered by the patient independently and the description of the first management data aggregate confirmed mutually between the prescribing doctor and the patient is judged (Step 207). In the case of the presence of the difference, the fact is printed in the space of Form 27-4 to give an indication (Step 208).
In data in the received first management data aggregate, defectiveness of description of data which is not covered by comparison with the data of the questionnaire (whether the result of a pregnancy test is described, etc.) and the presence or absence of an inappropriate answer and the like are judged by verification of predetermined data (standard answer) needed for each patient included in a patient group, e.g., the groups of women who may become pregnant, women without the possibility of becoming pregnant, and men (step 209). In the case of the presence of the defectiveness, the fact is similarly printed in the space to give an indication (Step 210).
It is judged whether or not data such as the quantity of a medicine to be prescribed has a problem such as description defectiveness or excess of a quantity (Step 211). In the case of the presence of the problem, the fact is similarly printed in the space to give an indication (Step 212).

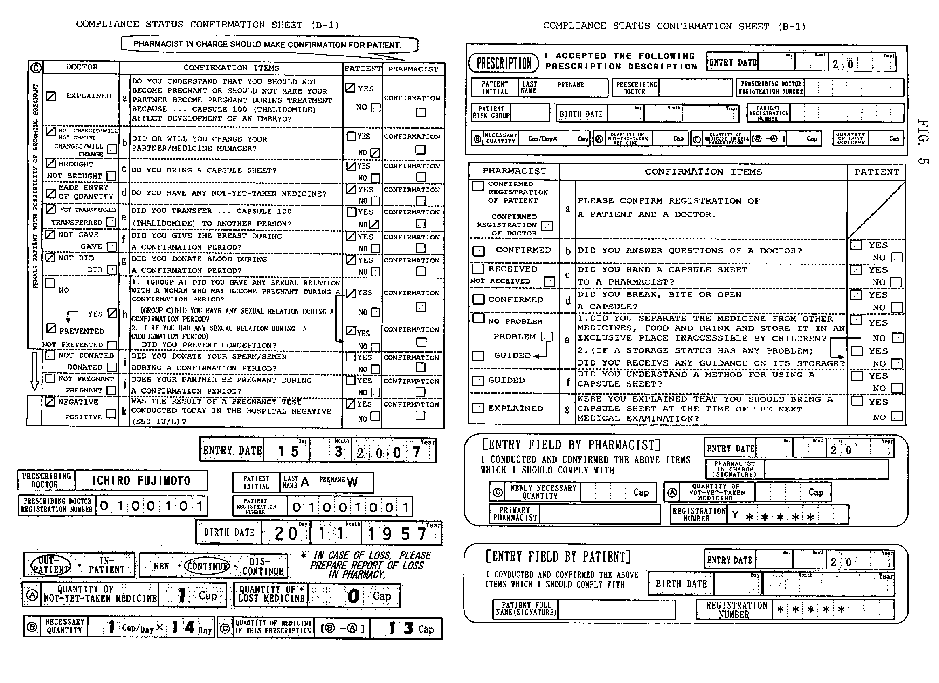

Finally, it is judged whether or not an interval between the latest date of obtaining data and the date of obtaining this data by the prescribing doctor/patient, i.e., a medicine prescription interval, is within a specified range (Step 213). In the case of out of the specified range, the fact is printed in the space to give an indication (Step 214).
When there is no problem in all judgment items, an integrated confirmation format (Form 27-2: Figure 5), in which a second confirmation format including a plurality of confirmation items between the pharmacist and the patient and check columns corresponding thereto is appended in addition to the first management data aggregate, is made. The integrated confirmation format is transmitted to the prescribing doctor by fax (Step 215), and the data is stored in an information storage medium (Step 216). In addition, in each step, when an indication of such defectiveness or such a problem is given, and when such indications are further observed in a plurality of parts, Form 27-4 in which all the facts are described is transmitted to the prescribing doctor by fax (Step 217).
When a pharmacist registered in the system belongs to the same medical institution as that to which a prescribing doctor belongs, the integrated confirmation format (Form 27-2: Figure 5) may be directly transmitted to the pharmacist by fax instead of being transmitted to the prescribing doctor. The pharmacist can report the fact to the prescribing doctor to decrease the effort of the doctor to obtain the integrated confirmation format from the management center.

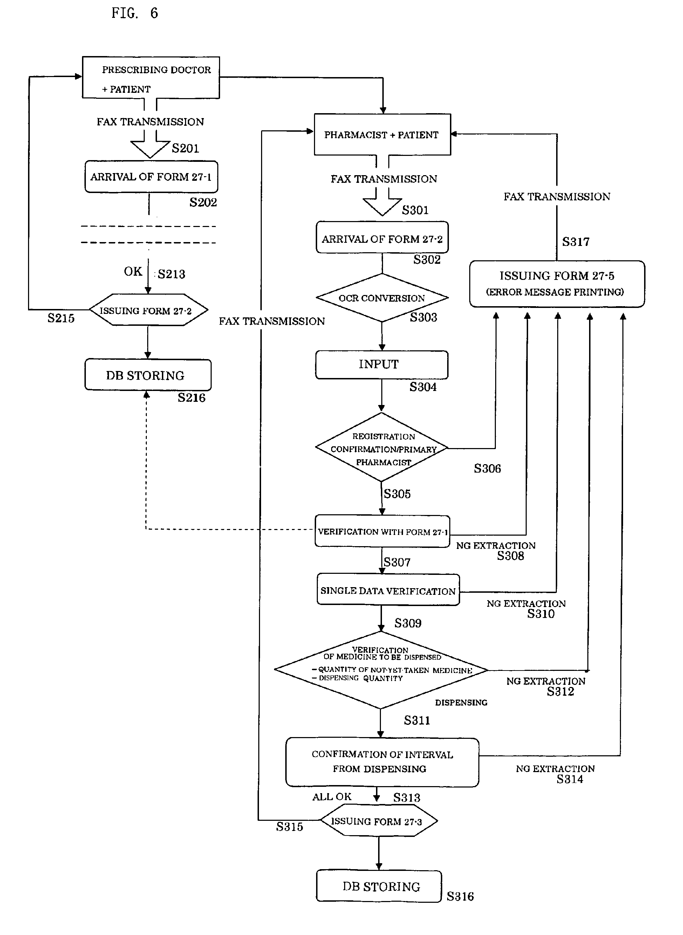

### (At pharmacy, see Figure 6)

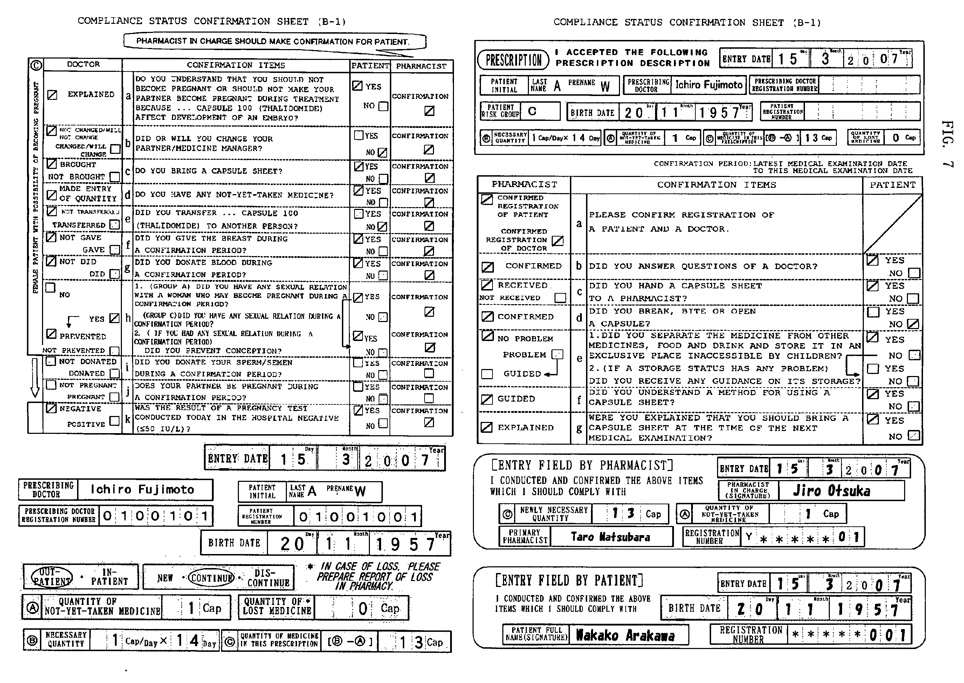

The pharmacist conducts a hearing (monitoring), from the patient, on a confirmation operation between the prescribing doctor and the patient using the integrated confirmation format (in which the second confirmation format is appended) obtained from the patient via the prescribing doctor, to confirm the state of implementation thereof. This operation may be performed by a pharmacist in charge who performs the task under the supervision of the primary pharmacist. As the container for exclusive use of a medicine (hereinafter may be referred to as "capsule sheet"), for example, a capsule sheet disclosed in Japanese Patent Laid-Open No. 2006-285973 may be used. This is a capsule sheet composed so that long rectangular thick paper is composed so as to be able to be folded in four, a plurality of holes in which a plurality of concaves of a PTP sheet in which thalidomide capsules of 2-week unit (14 days) are received are fitted are opened in a pair of left constitutive pieces, and the thick paper can be pasted together to be sealed. Use of the container allows confirmation of the quantity of a remaining medicine and surer confirmation of the above-mentioned defectiveness history by an interview with the patient while referring to the description written by the patient in a column for the state of taking a medicine and a comment column arranged in a pair of right constitutive pieces in which the PTP sheet is not sealed. After the confirmation, the pharmacist conducts compliance instructions and explains items which the patient should comply with, so that mutual confirmation prior to dispensing is carried out.
The patient makes entry of an entry date, a patient registration number and a patient birth date in the second confirmation format and signs in a signature column. The pharmacist makes entry of the quantity of a newly necessary medicine, the quantity of a not-yet-taken medicine, an entry date, a primary pharmacist name and a primary pharmacist registration number and signs in a signature column. In such a manner, the integrated confirmation format (Form 27-2; Figure 7), in which the second management data aggregate in which an answer between the pharmacist and the patient in the second confirmation format is recorded is appended, is transmitted to the management center via fax by the pharmacist (Step 301).

### (Judgment)

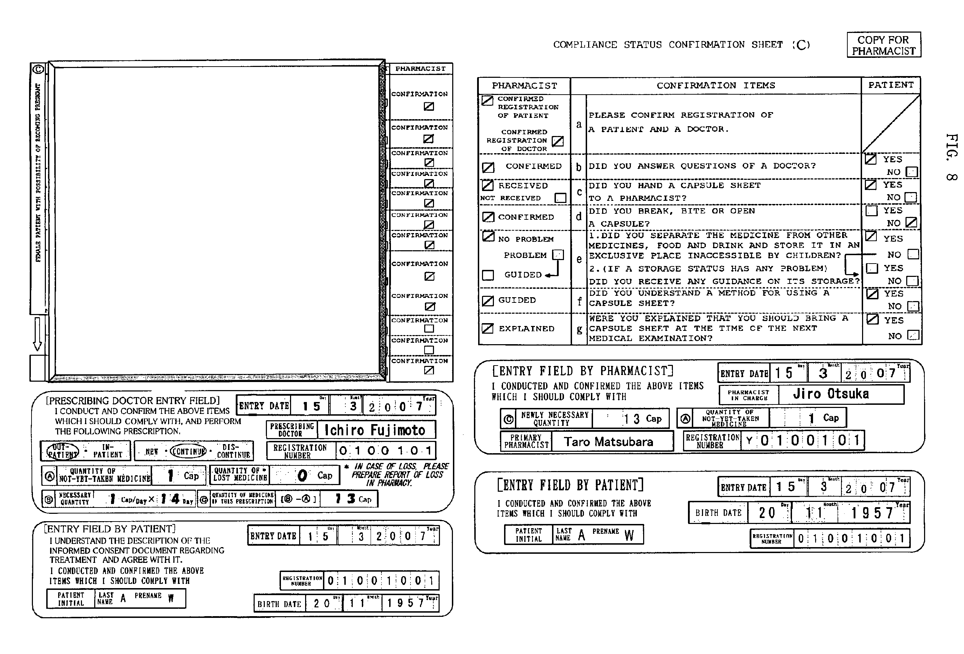

The management center uses OCR to convert a received integrated confirmation format into data (Step 304). It is judged whether or not a primary pharmacist in the converted second management data aggregate is registered (Step 305). In the case of an unregistered pharmacist, the fact is printed in the printing space of the left column of Form 27-5 (Figure 8) to give an indication (Step 306). It is also judged whether a patient is identical as a patient in the first management data aggregate.
Verification (pairing) with data in the first management data aggregate (Form 27-1) stored previously in an information storage medium is performed (Step 307). In the case of the absence of applicable data in the first management data aggregate, the fact is printed in the space of Form 27-5 to give an indication (Step 308).
It is verified and distinguished with needed predetermined data whether or not single data in the received second management data aggregate (Form 27-2) has description defectiveness, an inappropriate answer, etc. (Step 309). In the case of observing the defectiveness by verification, the fact is similarly printed in the printing space to give an indication (Step 310).

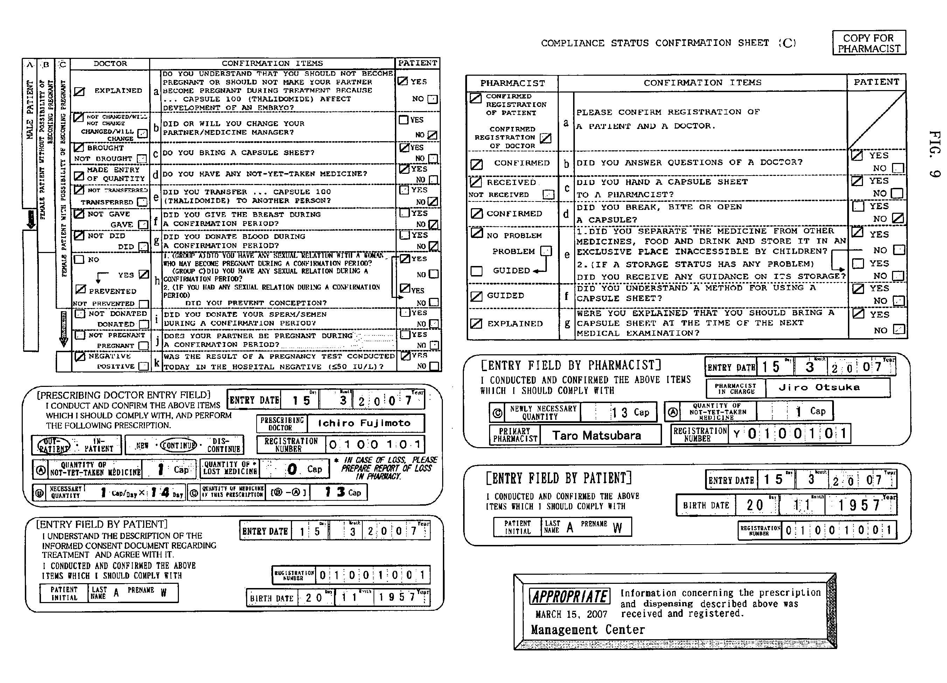

It is judged whether or not the data of the quantity of a dispensed medicine in the second management data aggregate conforms to prescription data in the first management data aggregate. More specifically, it is judged whether or not data such as the quantity of a medicine to be dispensed has a problem such as description defectiveness or excess of a quantity (Step 311). In the case of the presence of the problem, the fact is similarly printed to give an indication (Step 312).
Finally, it is judged whether or not an interval between a prescription date and the medical examination date is within an appropriate range (Step 313). In the case of out of the specified range, the fact is similarly printed to give an indication (Step 314).
When there is no problem in all judgment items, two pieces of Form 27-3 (Figure 9) showing the judgment results of dispensing conformity are transmitted to the pharmacist and the patient (Step 315), and the data is stored in an information storage medium (Step 316). In addition, in each step, when an indication of such defectiveness or such a problem is given, and when such indications are further observed in a plurality of parts, Form 27-5 in which all the facts are described is transmitted to the pharmacist (Step 317).

### [Embodiment 2]

In accordance with another embodiment of the present invention, the case of making entry of first and second confirmation formats made to be digital paper using a digital pen to perform transmission to and reception from a doctor or a pharmacist is described.
Each predetermined format shown in the questionnaire (Figure 2)and the integration format (Figure 5) including the first confirmation format (Figure 3) and the second confirmation format is printed on digital paper having a special dot pattern showing writing position information described in Japanese Patent Laid-Open Nos. 2005-141537 and 2006-178839 as described above. Although the dot pattern is invisible by a human, it is printed with carbon ink or the like which is readable by a digital pen, and the digital pen can detect an absolute position on the paper by reading the pattern.
In an input format, for example, items about the presence or absence of explanation about a side effect by the doctor, the level of understanding of the explanation by the patient, the presence or absence of a sexual relation and contraception of the patient during a confirmation period, the presence or absence of medical examination of a pregnancy test on the confirmation day, the test results, and the presence or absence of sperm donation during the confirmation period, as well as check boxes for a doctor and a patient, are printed in a confirmation column by a prescribing doctor and a patient on digital paper, so as to enable input of a mutual confirmation check for items which a prescribing doctor and a patient and/or a pharmacist and a patient should comply with in order to avoid the side effect of a medicine which may cause a serious side effect. The areas of the respective check boxes of the input format as well as an entry start check column and an entry termination check column are stored as a coordinate range on a pattern surface in a terminal.

A doctor and a patient mark respective check columns for a doctor and a patient using a digital pen while at a meeting mutually confirming each item of a compliance status confirmation sheet (first confirmation format) in which a plurality of confirmation items which the doctor and the patient should comply with are printed previously on digital paper to safely prescribe a medicine which may cause a serious side effect prior to prescription every time. The doctor and the patient who have marked all the check columns sign in respective signature columns using an electronic pen. If both check columns do not accord, the doctor cannot prescribe the medicine. If the doctor makes the mistake of marking "acceptance" in the check column in the prescription acceptance or rejection column of the confirmation sheet although any of the confirmation items is not processed, the server can also send an alert to the electronic pen.
A confirmation sheet is directly converted into digital data with a digital pen by completing entry of digital paper displaying a format which allows an input in such a manner using the digital pen and is transmitted to the server.

A predetermined format corresponding to the result of comparative judgment of necessary items for transmitted data is replied to a doctor, and therefore a pharmacist and a patient mark respective check columns for a pharmacist and a patient using a digital pen while mutually confirming each item of a compliance status confirmation sheet between a pharmacist and a patient (second confirmation format) in which a plurality of confirmation items which they should comply with are printed on digital paper prior to dispensing every time. The pharmacist and the patient who have marked all the check columns sign in respective signature columns using the digital pen. If both check columns do not accord, the pharmacist cannot dispense the medicine.
The transmitted data is verified with the data transmitted by the doctor, the necessary items are comparatively judged, and the results are replied to the pharmacist.

### [Embodiment 3]

Another embodiment, in which transmission and reception by a doctor or a pharmacist in accordance with the present invention are performed via a facsimile, is described below.

### (Prior to medical examination)

Processing of a questionnaire prior to medical examination is similar to that in Embodiment 1.

### (In consultation room)

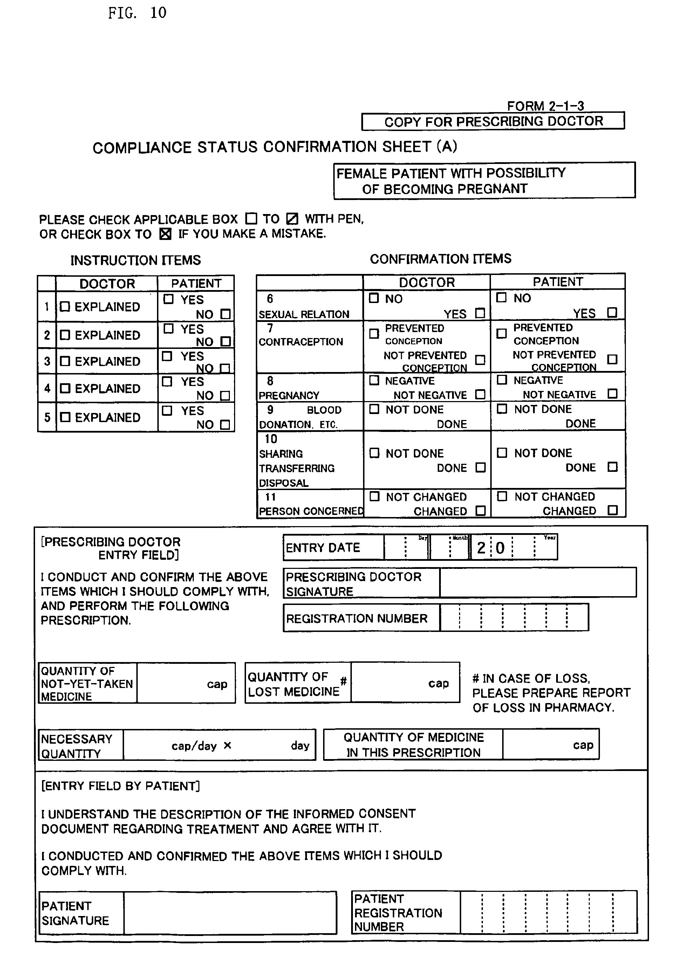

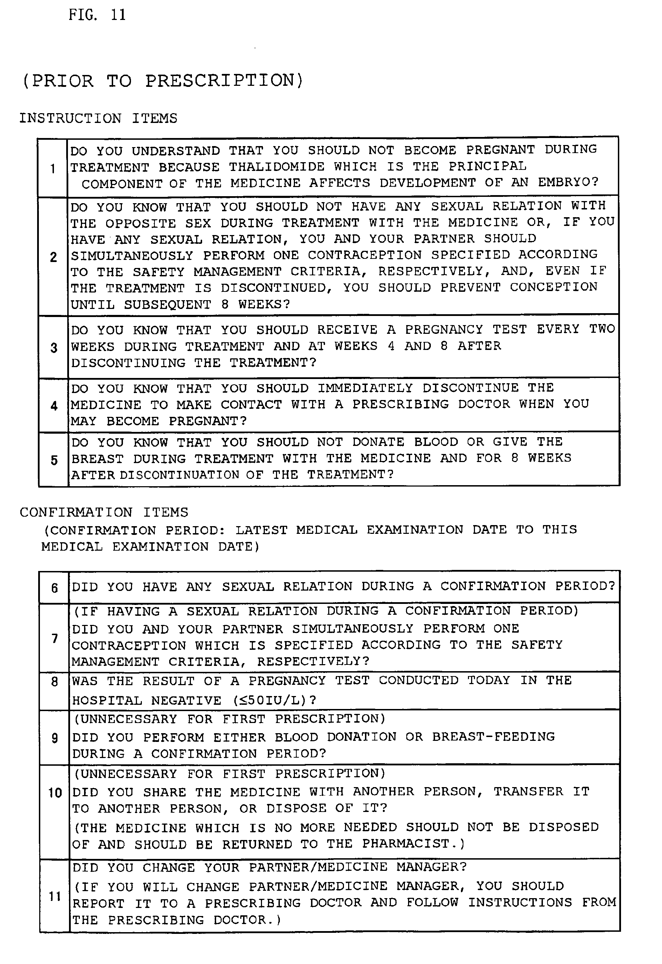

A first confirmation format (Form 2-1-3, Figure 10) in this embodiment is a format for a female patient group having the possibility of becoming pregnant. In this format, which includes the plurality of confirmation items between a doctor and a patient and check columns corresponding thereto, only the numbers of the respective items (instruction items 1-5, confirmation items (narrow sense) 6-11) or respective numbers and the simple keywords thereof (for example, "sexual relation", "contraception", etc.) are described as the confirmation items. In the confirmation items, for detailed descriptions, for example, the risk of a medicine, the time of a pregnancy test, and a coping process for emergency, etc., which are instruction items; and the presence or absence of a sexual relation, a contraception method in the case of the presence of the sexual relation, the result of a pregnancy test, and sharing or transferring of a medicine with another person, etc., which are confirmation items (narrow sense), item numbers 1-11 corresponding to the respective item numbers are given, and are attached as a confirmation item list (Figure 11) in an attached sheet. A patient and a prescribing doctor perform the mutual confirmation of the respective confirmation items (including instruction items) 1-11 referring to the confirmation item list (prior to prescription) at a meeting every time.

The patient makes entry of her patient registration number in the confirmation format and signs in a signature column. The prescribing doctor makes entry of the quantity of a medicine necessary until the next medical examination, the quantity of a lost medicine in the case of occurrence of loss of the medicine, the quantity of a not-yet-taken medicine, the quantity of this prescribed medicine taken into account the quantity of the medicine necessary until the next medical examination and the quantity of the not-yet-taken medicine, an entry date and a prescribing doctor registration number, and signs in a signature column (hereinafter, the respective items and the prescribing doctor name are referred together to as a prescription description). The prescribing doctor transmits a first management data aggregate, in which answers are recorded and written in such a manner, to the management center (Step 201), and subsequently performs processes up to Step 214 as in Embodiment 1.

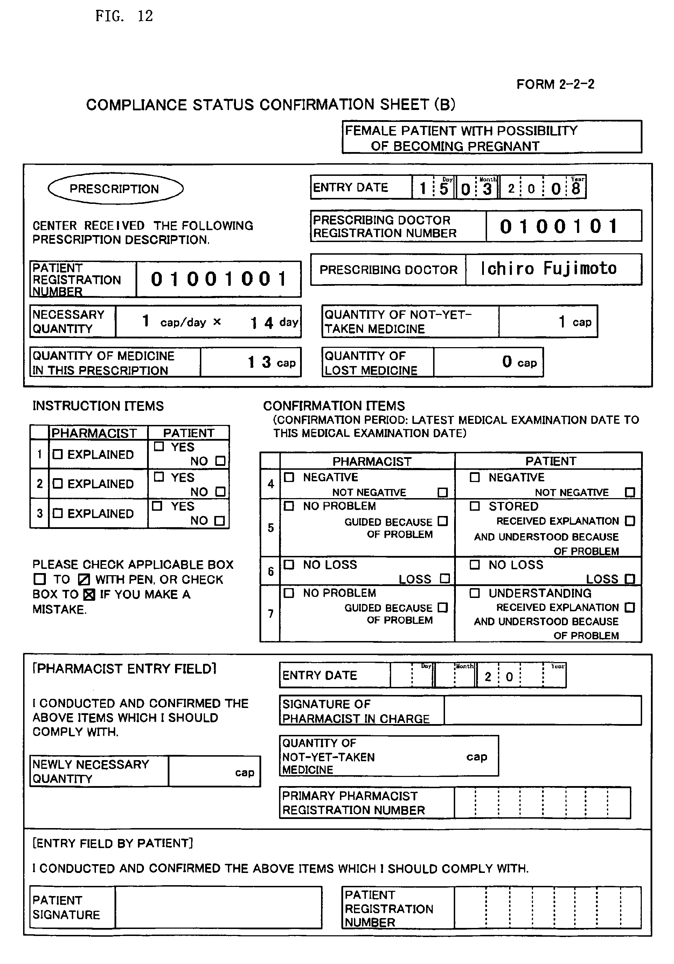

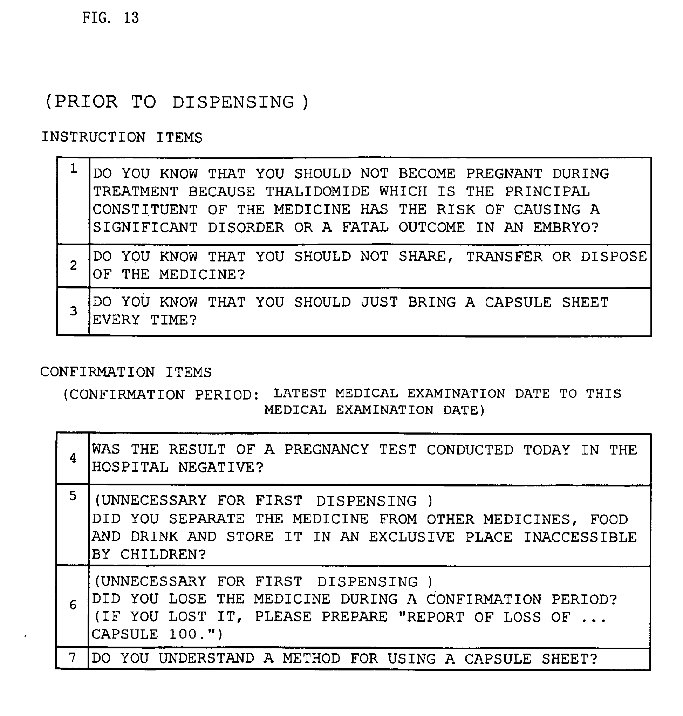

When there is no problem in all judgment items, an integrated confirmation format (Form 2-2-2: Figure 12), in which a second confirmation format including a plurality of confirmation items between the pharmacist and the patient and check columns corresponding thereto is appended in addition to the prescription description recorded by the doctor, which is data selected from a first management data aggregate (prescribing doctor name is automatically printed based on a prescribing doctor registration number), is made. In the second confirmation format, the numbers of the respective items (instructions items 1-3, confirmation items (narrow sense) 4-7) and check columns corresponding thereto are also described for the confirmation items, as in case of the first confirmation format. In the confirmation items, for detailed descriptions, for example, the risk of a medicine, and sharing or transferring of a medicine with another person, etc., which are instruction items; and the result of a pregnancy test, the presence or absence of a storage area for exclusive use of a medicine, and the presence or absence of loss of a medicine, etc., which are confirmation items (narrow sense), item numbers 1-7 corresponding to the respective item numbers of the second format are given, and are attached as a confirmation item list (Figure 13) in an attached sheet.
The integrated confirmation format, as in Embodiment 1, is transmitted to the prescribing doctor by fax (Step 215), and the data is stored in an information storage medium (Step 216).
When a pharmacist registered in the system belongs to the same medical institution as that to which the prescribing doctor belongs, the integrated confirmation format may be directly transmitted to the pharmacist by fax as in Embodiment 1.

### (At pharmacy)

A pharmacist conducts compliance instructions, explains items which the patient should complies with, and performs mutual confirmation every time prior to the dispensing according to the respective confirmation items 1-7 (including instruction items) referring to the confirmation item list (prior to dispensing) at a meeting with the patient using an integrated confirmation format obtained directly by fax-transmission from the patient via the prescribing doctor or from the management center.
The patient makes entry of her patient registration number in the integrated confirmation format and signs in a signature column, and the pharmacist makes entry of the quantity of a newly necessary medicine, the quantity of a not-yet-taken medicine, an entry date and a primary pharmacist registration number, and signs in a signature column. As in Embodiment 1, an integrated confirmation format (Form 2-2-2: Figure 12), in which a second management data aggregate in which answers between the pharmacist and the patient are recorded is appended, is transmitted to the management center via fax by the pharmacist (Step 301).

### (Judgment)

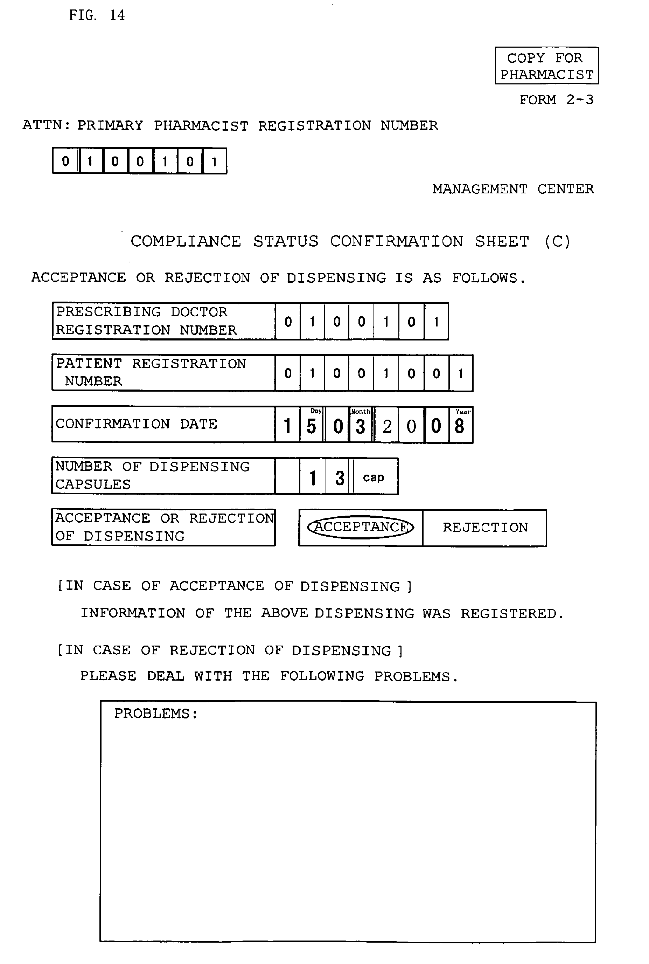

The management center performs Steps 304 to 314 by processing a received integrated confirmation format as in Embodiment 1. When there is no problem in all judgment items, a confirmation sheet (Form 2-3: Figure 14) showing a prescribing doctor registration number, a patient registration number, a confirmation date, the number of dispensed medicines and the result with respect to acceptance or rejection of the dispensing is transmitted to a pharmacist (Step 315), and the data is stored in an information storage medium (Step 316). When an indication of defectiveness or a problem in a judgment item is given, and when problems are further observed in a plurality of parts, a confirmation sheet (Form 2-3: Figure 14) in which all the facts are described in a printing space in a lower section is transmitted to the pharmacist (Step 317).

### Brief Description of the Drawings

Figure 1 is a flow chart describing (prior to medical examination) and (in consultation room) in accordance with embodiments of the present invention;
Figure 2 is a view representing a questionnaire (Form 26-1) used by a patient in accordance with the present invention;
Figure 3 is a view representing a first confirmation format (Form 27-1) used in accordance with the present invention;
Figure 4 is a view representing a format (Form 27-4) replied to a doctor when a first management data aggregate is not appropriate;
Figure 5 is a view representing an integrated confirmation format (Form 27-2 prior to fax transmission) which is replied to a doctor and used between a pharmacist and a patient;
Figure 6 is a flow chart describing (at a pharmacy) in accordance with embodiments of the present invention;
Figure 7 is a view representing an integrated confirmation format (Form 27-2 after reception of fax) in which a second management data aggregate is recorded;
Figure 8 is a view representing an integrated confirmation format (Form 27-5) replied when the first and second management data aggregates in the integrated confirmation format are not appropriate;
Figure 9 is a view representing an integrated confirmation format (Form 27-3) replied when the first and second management data aggregates in the integrated confirmation format are appropriate;
Figure 10 is a view representing a first confirmation format (Form 2-1-3) used in Embodiment 3;
Figure 11 is a confirmation item list composing an attached sheet of the first confirmation format used in Embodiment 3;
Figure 12 is an integrated confirmation format used between a pharmacist and a patient in Embodiment 3;
Figure 13 is a confirmation item list composing an attached sheet of the integrated confirmation format used in Embodiment 3; and
Figure 14 is a confirmation sheet used in Embodiment 3.

## Claims

1. A medicine management system comprising input means, distinction means, transmitting means, and an information storage medium as described in the following (1) to (6):
(1) input means for inputting a first management data aggregate including a first confirmation format including a plurality of confirmation items between a doctor and a patient for avoiding a serious side effect to a medicine and check columns corresponding thereto; and answers recorded therein, in response to transmission from a doctor;
(2) distinction means (i) to (iii) described below for data in the first management data aggregate:
(i) distinction means for distinguishing whether or not a doctor and a patient are persons registered previously in an information storage medium for dealing with said medicine;
(ii) distinction means for distinguishing whether or not each piece of patient data conforms to a medicine prescription requirement according to a patient group; and
(iii) distinction means for distinguishing whether or not medicine prescription interval and prescribed medicine quantity data is within an appropriate numerical range;
(3) transmitting means for producing an integrated confirmation format in which a second confirmation format including a plurality of confirmation items between a pharmacist and a patient and check columns corresponding thereto is appended in addition to the first management data aggregate or in addition to a prescription description recorded by a doctor, which is the data selected from the first management data aggregate, only in case of distinction as conformance in the distinction according to (2), and replying the integrated confirmation format to the doctor or the pharmacist;
(4) input means for inputting an integrated confirmation format, in which a second management data aggregate including a recorded answer between a pharmacist and a patient in the second confirmation format is appended, in response to transmission from the pharmacist;
(5) distinction means (i) to (iii) described below for data in the second management data aggregate:
(i) distinction means for distinguishing whether or not a patient in the second management data aggregate is the same as a patient in the first management data aggregate;
(ii) distinction means for distinguishing whether or not a pharmacist is a person registered previously in an information storage medium for dealing with said medicine; and
(iii) distinction means for distinguishing whether or not dispensing quantity data in the second management data aggregate conforms to prescription data in the first management data aggregate; and
(6) transmitting means for replying judgment of the propriety of dispensing depending on the distinction result to the pharmacist.

2. The medicine management system according to claim 1, wherein data obtained by the input means is character data obtained by OCR-processing the first or second management data aggregate transmitted via a facsimile by a doctor or a pharmacist.

3. The medicine management system according to claim 1, wherein data obtained by the input means is data written in the first or second confirmation format made to be digital paper by at least one of a doctor, a pharmacist and a patient using a digital pen.

4. The medicine management system according to any of claims 1 to 3, wherein check columns for monitoring the plurality of confirmation items performed mutually by the doctor and the patient as confirmation items for the pharmacist and the patient are included in the integrated confirmation format replied to the doctor or the pharmacist when the first management data aggregate is appropriate, and the integrated confirmation format is stored in the information storage medium.

5. The medicine management system according to any of claims 1 to 3, wherein a predetermined format corresponding to a judgment result of appropriate prescription and dispensing is made, replied to the pharmacist and stored in the information storage medium.

6. The medicine management system according to any of claims 1 to 3, wherein, when it is judged that prescription and dispensing are not appropriate due to defectiveness of the input data, the predetermined format which is further provided with a printing space for indicating the defectiveness is made, and appropriate data is retransmitted by transmitting the indication to the doctor and/or the pharmacist.

7. The medicine management system according to any of claims 1 to 3, further comprising: comparing data obtained by the input means based on the first confirmation format transmitted by the doctor with patient information stored and registered in the information storage medium based on a questionnaire obtained previously from the patient.

8. The medicine management system according to any of claims 1 to 3, wherein the first management data aggregate includes a plurality of items selected from items about the presence or absence of explanation about the side effect by the doctor, the level of understanding of the explanation by the patient, the presence or absence of transfer of the medicine to a third person, the presence or absence of a sexual relation and contraception of the patient during a confirmation period, the presence or absence of medical examination of a pregnancy test on a confirmation day, the test results, and the presence or absence of sperm donation during the confirmation period as confirmation items for the doctor and the patient; and answers therefor.

9. The medicine management system according to claim 1, wherein the integrated confirmation format replied to the doctor or the pharmacist further includes any of items about the presence or absence of an answer for a question of the doctor, a storage status of the medicine, and use and recovery of a container for exclusive use of the medicine as confirmation items for the pharmacist and the patient.

10. The medicine management system according to claim 9, wherein the integrated confirmation format replied to the doctor or the pharmacist further includes items about a quantity of a not-yet-taken medicine and a prescription or dispensing quantity.

11. The medicine management system according to any of claims 1 to 10, further comprising respective signature columns for a doctor, a patient and/or a pharmacist.

12. The medicine management system according to any of claims 1 to 11, wherein the first and second confirmation formats are separated into (i) a section comprising codes indicating respective confirmation items and check columns corresponding thereto; and (ii) an attached sheet section indicating detailed descriptions of the confirmation items corresponding to the codes.
